# EUROPEAN PATENT SPECIFICATION

(11) **EP 3 891 505 B1**
(45) Date of publication and mention of the grant of the patent: **30.11.2022**
(21) Application number: 20701436.6
(22) Date of filing: 20.01.2020
(51) Int. Cl.: G01N 33/60, G01N 33/96

(54) **A METHOD FOR MEASURING A CONCENTRATION OF A BIOMARKER/ANALYTE IN BLOOD FROM MAMMALS**
VERFAHREN ZUR MESSUNG DER KONZENTRATION EINES BIOMARKERS/ANALYTEN IN BLUT VON SÄUGETIEREN
MÉTHODE DE MESURE D'UNE CONCENTRATION D'UN BIOMARQUEUR/ANALYTE DANS LE SANG DE MAMMIFÈRES

(30) Priority: 28.01.2019 SE 1950094
(43) Date of publication of application: 13.10.2021
(73) Proprietor: REDHOTDIAGNOSTICS AB, 15136 Södertälje (SE)
(72) Inventor: REHNMARK, Stefan, 15 136 Södertälje (SE); RANDAHL, Håkan, 151 36 Södertälje (SE)
(74) Representative: Swea IP Law AB
(86) International application number: PCT/EP2020/051233
(87) International publication number: WO 2020/156849

(56) References cited:
- WO-A1-2014/178787
- WO-A1-2017/210218
- US-A1- 2002 019 056
- US-A1- 2016 025 709
- US-B2- 10 067 140
- ADAWAY JOANNE E ET AL: "Therapeutic drug monitoring and LC-MS/MS", JOURNAL OF CHROMATOGRAPHY B: BIOMEDICAL SCIENCES & APPLICATIONS, vol. 883, 29 September 2011 (2011-09-29), pages 33-49, XP028889178, ISSN: 1570-0232, DOI: 10.1016/J.JCHROMB.2011.09.041

## Description

### Technical field

The present invention relates to a method for measuring a concentration of one or more biomarker in blood from a mammal comprising providing a kit of parts for sampling of blood, distributing the kit to a location of the mammal, receiving the vial comprising a blood sample from the mammal inserted into the vial, analysing the blood sample to determine the quality of the sample and the concentration of the one or more biomarker in the blood of the mammal, by centrifuging the vial and performing a direct analysis of the supernatant.

### Background

Personalizing medication or Tailor Dose Monitoring (TDM) of a patient and control for substance abuse requires frequent measuring of biomarkers in blood of a mammal, such as a patient or a drug addict. It is most convenient if the blood samples can be taken in a home environment without the need for professional health care personal. However, analyzing blood samples taken in a home environment is challenging, when sampling is performed by a layman and then transported to a laboratory for analysis. Several methods for quantification or qualification of a biomarker in a blood sample taken by a layman at home have been developed.

The most common method for blood sampling that exist today are so-called dry blood spot analysis, which means that blood drawn from a lancet stick in the finger is placed on a filter disc. In general blood, has to be placed on four different filter discs. The filters discs has to be dried to prevent the blood from spreading to surrounding objects. When the discs arrive at the laboratory, they are visually inspected to ensure that they are correctly filled with blood. Thereafter, the discs are extracted by a solvent and the eluate is analyzed. An extraction will never be 100%, and a validation has to be made to determine the amount of the compound/biomarker that sticks to the filter. There are no internal controls that can give an indication about how the filter discs have been treated during the sampling and transportation or about how much of the biomarker has been extracted. Furthermore, during the extraction, the blood sample will be diluted, which places higher demands on the analytic equipment and increases risks for errors in the analyzed results.

In all mass spectroscopy (MS), or LC-MS/MS methods, one or more internal standard are used as a control to ensure that the sample has undergone the process, from preparation of the sample to completed analysis, in a correct manner. Any differences are compensated for by the added internal standard. It would therefore be advantageous to use one or more internal standards early in the method. This would allow for a better control of the quality of the analysis.

US2016/0025709 discloses a kit that can be used for qualification of damaged DNA. The blood from a mammal is collected in a capillary that is put into a vial. The vial is analyzed in the laboratory. The vial does not comprise an internal standard(s) and therefore does not allow for direct analysis of a quality and concentration of a biomarker in blood of a mammal.

WO2017210218 discloses a device that may be used for collecting a blood sample, whereby a volume of blood is collected in a vial that contains desiccant beads to dry the sample prior to analysing the sample in a robotic sample processing system. Internal standard(s) are not used in this device and neither does a dried blood sampling allow for direct measurement of both quality and concentration of a biomarker.

US10067140 discloses a system for determining a concentration of a biomarker in a blood sample. The method comprises collecting blood, adding an internal standard, filtering the sample and then analysing the sample in a mass spectroscope (MS). A special device for filtering the blood is also disclosed. No kit comprising a vial with one or more internal standard is disclosed. Therefore, this system does not allow for direct quantification and qualification of a blood sample from a patient, whereby the blood sample has been transported from the patient's home to a laboratory.

Adaway JE, et al. Therapeutic drug monitoring and LC-MS/MS, J. Chromatogr. B. 2012, 883-884, 33-49, disclose a method for monitoring therapeutic drugs using LC-MS/MS. The article explains the difficulty of removing proteins and lipids from a blood sample prior to analysis. Many internal standards that can be used in the analysis are mentioned. There is no mention of a kit comprising a vial with one or more internal standard. Neither is explained how an LC-MS/MS method can be used for direct quantification and qualification of a blood sample from a patient, whereby the blood sample has been transported from the patient's home to a laboratory.

US2002019056 discloses a method for analysing dicarboxylic acids in a blood sample using esterification of the acid. The method can be used for diagnosing vitamin B12 deficiencies.

WO2014178787 discloses novel phophatidylalkanols that may be used as internal standards for measuring long-term alcohol consumption. There is no mention of a kit comprising a vial with one or more internal standard, such that a direct assessment of a quality and concentration of an amount of alcohol in a blood sample is possible.

There is a demand from the health care sector for methods that can be used in a home environment and that are simple and cost-effective. The methods available do not fulfill the requirements and have not penetrated the market. There are no methods on the market today that guaranties both assessment of quality of the sample as well as giving a good determination of the blood concentrations of the biomarker.

None of the known methods allow for normalization of the obtained results.

### Summary

The aim of the present invention is to overcome the above-mentioned problems and to provide an improved method for determining the quality of a sample and the concentration of a biomarker in blood of a mammal.

The method comprises a method for measuring a concentration of one or more biomarker/analyte in blood from a mammal comprising:
- providing a kit of parts for sampling blood, the kit of parts comprising a lancet and a capillary adapted to sample a defined volume of blood from the mammal and a vial comprising an extraction fluid together with one or more internal standard, wherein the one or more internal standard is a radioisotope of one or more biomarker selected from a group comprising one or more of 2H, 3H, 11C, 13C, 14C, 13N, 15N, 150, 170 and 180, the radioisotope of said one or more biomarker being adapted to assess a quality and a concentration of one or more biomarkers in the blood sample,
- distributing the kit of parts to a location of the mammal,
- receiving the vial comprising a blood sample from the mammal, inserted into the vial,
- analysing the blood sample to determine the quality of the sample and the concentration of the one or more biomarkers in the blood of the mammal by centrifuging the vial and performing a direct analysis of the supernatant, and normalizing the analyzed results between different samples and a standard curve.

In an aspect, the one or more internal standard is a 2H, 3H, 11C, 13C and/or 14C radioisotope of the one or more biomarker adapted to assess quality and concentration of the one or more biomarker in the blood sample. In one aspect, the one or more internal standard is a 13C and/or 2H radioisotope of the one or more biomarker. In an aspect, the one or more internal standard is a 13C and 2H radioisotope of the one or more biomarker. One 13C and 2H radioisotope of the one biomarker may be used to increase the mass difference between the internal standard and the biomarker. This may improve the quality of the analysis. In another aspect, the one or more internal standard is a 2H radioisotope of the one or more biomarker. In an aspect, the one or more internal standard is a 13C radioisotope of the one or more biomarker. Such internal standards can be manufactured and can be used in such low concentrations that there is no risk for injury by radiation. An advantage of the new method is that the blood sampling can be performed by a layman at home. No trained health personal is needed. This saves time and money for the health care sector as well as for the person that is to be monitored. Due to the presence of the one or more internal standard, a direct control of the quality of the blood sample is possible. In the method of the invention, both quality and concentration of the one or more biomarker in the blood sample can be determined.

In one aspect, the extraction fluid is a solution adapted for use in the analysis method, such as mass spectroscopy (MS). A further advantage of the method is that there is no need for an additional extraction step prior to analysis for removal of a solvent. Direct analysis of the supernatant simplifies the method and saves costs and time for analysis. This also allows the method to be used in an automated setting. "Direct analysis" may include a method as defined above which optionally comprises an additional extraction step prior to analysis in the analytical instrument. Preferably, the method with the additional extraction step can still be used in an automated setting. The extraction fluid is optimized for the one or more particular biomarker. In general, the one or more biomarker should be stable for 14 days at room temperature.

One or more internal standard may be one internal standard or more than one internal standard, such as two or three internal standards. More than one internal standard may be used to measure both quality and concentration of one or more biomarker. In one aspect, two or three internal standards are used to measure both quality and concentration of two or three biomarkers. This allows for example for measurement of an amount of a particular pharmaceutical active compound (API) as well as its metabolites. In one aspect, one internal standard is used to measure both quality and concentration of one biomarker. The internal standard may have each individually have one or two radioisotopes. Thus, one or more biomarker comprising one or more radioisotope, includes one biomarker having one radioisotope, and one biomarker having two radioisotopes, which radioisotope may be the same or different, as well as two or more biomarkers having each one radioisotope, which radioisotope may be the same or different, and two or more biomarkers having each one or two radioisotopes, which radioisotope may be the same or different.

The analyzed results can thus be used as a basis for diagnosis and for giving the patient an adequate treatment and personalize the drug (API) prescription or to tailor dose monitoring. The volume of blood is determined by the volume of the capillary sampling device. The defined volume of blood may be from 10 to 100 µl or 25 to 75 µl, or 40 to 60 µl, or about 50 µl, or 25 µl, or 10 µl. In one aspect, the defined volume of blood is 50 µl.

During analysis, the one or more internal standard in the extraction fluid can be used to normalize the analyzed results between different samples and the standard curve. This gives an immediate feedback whether the sample has been treated correctly, from sampling and extraction, through the logistics chain, to the analysis of the sample in the laboratory. Normalization saves time and costs for analysis. The method thus provides a quality assurance after direct analysis of the supernatant. This feedback is important for correct diagnosis, control of levels of the API or control of drug abuse. This is especially important for APIs, wherein the difference between therapeutic and toxic levels is relatively small and monitoring of concentration of the amount of API in blood of a patient is important to prevent adverse effect of the API.

The method of the invention is simple, accurate in quality and concentration assessment and cost effective.

In one aspect, the method comprises (not claimed):
- providing a kit of parts for sampling blood, the kit of parts comprising a lancet and a capillary adapted to sample a defined volume of blood from the mammal and a vial comprising an extraction fluid together with one or more internal standard, wherein the one or more internal standard is a radioisotope of one or more biomarker selected from a group comprising one or more of 2H 3H, 11C, 13C, 14C, 13N, 15N, 150, 170 and 180, the radioisotope of said one or more biomarker being adapted to assess a quality and a concentration of one or more biomarkers in the blood sample,
- distributing the kit of parts to a location of the mammal,
- taking a blood sample from the mammal by creating a wound with the lancet,
- collecting a defined volume of the blood in the capillary,
- entering the blood sample or the capillary into the vial,
- receiving the vial comprising the blood sample from the mammal inserted into the vial,
- optionally, extracting the extraction fluid from the vial,
- centrifuging the vial, and
- analysing the supernatant to determine the quality of the sample and the concentration of the one or more biomarker/analyte in the blood of the mammal.

In an aspect, the one or more internal standard is a 2H 3H, 11C, 13C and/or 14C radioisotope of the one or more biomarker. In one aspect, the one or more internal standard is a 13C and/or 2H radioisotope of the one or more biomarker. In an aspect, the one or more internal standard is a 13C and 2H radioisotope of the one or more biomarker. In another aspect, the one or more internal standard is a 2H radioisotope of the one or more biomarker. In an aspect, the one or more internal standard is a 13C radioisotope of the one or more biomarker. Such internal standards are relatively easy to manufacture and can be used in such low concentrations that there is no risk for injury by radiation.

In one aspect, one internal standard is used to measure both quality and concentration of one biomarker. In another aspect, the mammal is a human.

In a further aspect, the one or more internal standard is adapted for measuring a quality and concentration of one or more pharmaceutical active compound (API) that can be stabilized in an extraction fluid. In one aspect, the API is selected from the group comprising or consisting of antifungal API, antiviral API, immunodepression API, anticonvulsant API, antidepressant API, antibiotic API, anticancer API, vitamins, cardiovascular API, painkilling API and opiates. In another aspect, the API is selected from the group comprising or consisting of immunodepression API, anticonvulsant API, antidepressant API, anticancer API, vitamins, cardiovascular API, painkilling API and opiates. In an aspect, the API is selected from the group comprising or consisting of antidepressant API, anticancer API, vitamins, cardiovascular API, painkilling API and opiates. In a further aspect, the API is selected from the group comprising or consisting of antidepressant API, anticancer API, vitamins and cardiovascular API. In one aspect, the one or more internal standard is adapted for measuring quality and concentration of anticancer API.

In another aspect, the one or more internal standard is adapted for measuring a quality and concentration of one or more pharmaceutical active compound that can be stabilized in an extraction fluid, wherein the one or more internal standard is selected from the group comprising or consisting of tamoxifen, z-endoxifen, 4-hydroxytamoxifen, CPA-cyclophosphamide and its active metabolite PAM-hb, DOC-docetaxel, DOX-doxorubicine, PAC-paclitaxel, EPI-epirubicin, statins, steroids, such as testosterone, or vitamins, such as vitamin B or D, or compounds related to addictions, such as opioids, cocaine, heroin, fentanyl, cannabinoids, marijuana, benzodiazepines, hallucinogens and methamphetamine, codeine, ibuprofen, dihydrocodeine, morphine, dextropropxyphene napsylate, aminorex, lidocaine, iso-LSD, norcocaine, amitriptyline, pemoline, prazepam, imipramine, propafenone, pheniramine, chlorpromazine, amphetamine sulfate, lofexidine hydrochloride, clozapine, ecgonine methyl ester, diphenylhydramine, estazolam, 3,4-methylenedioxy-amphetamine, melatonin, doxepin, ketamine, mescaline, aprobarbital, buprenorphine, benzoylecgnine, trifluoperazine, methadone, ecgonine ethyl ester, midazolam, fentanyl, norketamine, chlordiazepoxide, caffeine, hydrocodone, fenfluramine, tramadol, lorazepam, phenylpropanolamine, flunitrazepam, amobarbital, flurazepam, phencyclidine (PCP), barbital, carbamazepine, vancomycin and phenobarbital.

In another aspect, the one or more internal standard is adapted for measuring a quality and concentration of one or more pharmaceutical active compound that can be stabilized in an extraction fluid, wherein the one or more internal standard is selected from the group comprising or consisting of tamoxifen, z-endoxifen and 4-hydroxytamoxifen. Especially in the case of tamoxifen, it is advantageous to use more than one internal standard to also measure active metabolites of tamoxifen in the blood sample. In one aspect, the internal standards are radioisotopes of tamoxifen, z-endoxifen and 4-hydroxytamoxifen. In another aspect, the internal standards are radioisotopes of tamoxifen and z-endoxifen. In a further aspect, the internal standards are radioisotopes of tamoxifen and 4-hydroxytamoxifen. In yet another aspect, the internal standards are radioisotopes of z-endoxifen and 4-hydroxytamoxifen. In one aspect, the internal standard is a radioisotope of tamoxifen. In a further aspect, the one or more internal standard is a 13C radioisotope of tamoxifen, z-endoxifen and/or 4-hydroxytamoxifen.

Tamoxifen is a pre-drug that will be metabolized to active substances, whereas z-endoxifen and 4-OH tamoxifen are the most active metabolites. The concentration of tamoxifen is measured to find out how much drug there is available and z-endoxifen and 4-OH tamoxifen are measured to determine that the patient has blood concentrations of this anti-cancer API within the narrow therapeutic window.

In another aspect, the one or more internal standard is one or more compounds related to addictions selected from the group comprising or consisting of opioids, cocaine, heroin, fentanyl, cannabinoids, marijuana, benzodiazepines, hallucinogens, methamphetamine, amphetamine, codeine, dihydrocodeine, phencyclidine (PCP), morphine, iso-LSD, norcocaine, fentanyl, methadone, hydrocodone and tramadol.

In a further aspect, the one or more internal standard is selected from the group comprising or consisting of vitamins, such as vitamin B or D. In a further aspect, the one or more internal standard is a radioisotope of methylmalonic acid adapted for measuring Vitamin B deficiency. In a further aspect, the one or more internal standard is a 2H or a 13C radioisotope of methylmalonic acid. In a further aspect, the one or more internal standard is a 2H and 13C radioisotope of methylmalonic acid. In yet a further aspect, the one or more internal standard is a radioisotope of dihydrotachysterol adapted for measuring Vitamin D deficiency. In a further aspect, the one or more internal standard is a 2H or a 13C radioisotope of dihydrotachysterol. In a further aspect, the one or more internal standard is a 2H and 13C radioisotope of dihydrotachysterol.

In another aspect, the one or more internal standard is selected from the group comprising or consisting of lorazepam, phenylpropanolamine, flunitrazepam, amobarbital, flurazepam, PCP, barbital, carbamazepine, chlordiazepoxide, aprobarbital, vancomycin and phenobarbital.

In a further aspect, the one or more internal standard is selected from the group comprising or consisting of CPA-cyclophosphamide and its active metabolite PAM-hb, or DOC-docetaxel, DOX-doxorubicine, PAC-paclitaxel and EPI-epirubicin.

In yet a further aspect, the one or more internal standard is selected from the group comprising or consisting of statins selected from the group comprising or consisting of atorvastatin, fluvastatin, cerivastatin, lovastatin, mevastatin, pitavastatin, pravastatin, rosuvastatin and simvastatin, or mixtures thereof. In an aspect, the internal standard is selected from the group comprising or consisting of pravastin, rosuvastatin, simvastatin and atorvastatin.

In yet another aspect, the one or more internal standard is selected from the group comprising or consisting of steroid hormones, such as glucocorticoids, mineralocorticoids, androgens, oestrogens and progestogens. In one aspect, the steroid hormones is selected from the group comprising or consisting of alclometasone, prednisone, dexamethasone, triamcinolone, cortisone, fludrocortisone, oxandrolone, oxabolone, testosterone, nandrolone, diethylstilbestrol (DES) and estradiol, norethisterone, medroxyprogesterone acetate, hydroxyprogesterone caproate, cyproterone acetate, mifepristone and gestrinone, or mixtures thereof. In an aspect, the internal standard is selected from the group comprising or consisting of aldosteron, androsteron, crotisol, progetsteron and testosteron, or mixtures thereof.

In one aspect, the one or more internal standard is an antidepressant API selected from the group comprising or consisting of selective serotonin reuptake inhibitors, serotonin-norepinephrine reuptake inhibitors, serotonin modulators and stimulators, serotonin antagonists and reuptake inhibitors, norepinephrine reuptake inhibitors, norepinephrinedopamine reuptake inhibitors, tricyclic antidepressants, tetracyclic antidepressants, monoamine oxidase inhibitors and NMDA receptor antagonists, or mixtures thereof.

In another aspect, the one or more internal standard is an antidepressant API selected from the group comprising or consisting of bupropiontrazodone, nefazodone, vilazodone, vortioxetine, mitriptyline, bupropion, citalopram, desvenlafaxine, duloxetine, escitalopram, fluoxetine, mirtazapine, nortriptyline, paroxetine, sertraline, trazodone and venlafaxine, or mixtures thereof. In an aspect, the internal standard is selected from the group comprising or consisting seratralin, citalpram, excitaopram, venlafaxin and flouxetin, or mixtures thereof.

In a further aspect, the one or more internal standard is selected from the group comprising or consisting of ibuprofen, dextropropxyphene napsylate, aminorex, lidocaine, amitriptyline, pemoline, prazepam, imipramine, propafenone, pheniramine, chlorpromazine, amphetamine sulfate, lofexidine hydrochloride, clozapine, ecgonine methyl ester, diphenylhydramine, estazolam, 3,4-methylenedioxy-amphetamine, melatonin, doxepin, ketamine, mescaline, buprenorphine, benzoylecgnine, trifluoperazine, ecgonine ethyl ester, midazolam, norketamine, caffeine and fenfluramine, or mixtures thereof.

In another aspect, the one or more internal standard is phosphatidylethanol 16:0/18:1 (PEth-16:0/18:1, PEth 16:0/18:2, PEth 18:1/16:0, and/or PEth 18:2/16:0)) adapted for measuring long-term alcohol consumption in a mammal, such as a human. In a further aspect, the one or more internal standard is a 2H radioisotope of phosphatidylethanol 16:0/18:1 (PEth-16:0/18:1, PEth 16:0/18:2, PEth 18:1/16:0 and/or PEth 18:2/16:0).

In a further aspect, the blood sample is analysed using mass spectrometry (MS), liquid chromatography- mass spectrometry (LC-MS, LC-MS/MS, gas chromatography (GC-MS) or GC-MS/MS. In one aspect, blood sample is analysed using mass spectrometry (MS).

In one aspect, the extraction fluid is selected from the group comprising 2-propanol, methanol and acetonitrile, formic acid, or mixtures thereof. The fluid is adapted to substantially stop all enzyme activity in the blood sample, stabilize the sample and extract the biomarker from the blood sample.

The disclosure also relates to a kit of parts (not claimed) comprising or consisting of a lancet and a capillary adapted to sample a defined volume of blood from the mammal and a vial comprising or consisting of an extraction fluid together with one or more internal standard for use in the method as defined anywhere herein. In an aspect, the kit is for use in diagnosing a disease. In one aspect, the disease is selected from the group comprising or consisting of addiction, such as alcoholism, a disease like cancer, such as breast cancer or prostate cancer, cardiovascular diseases, high blood pressure, viral, fungal or bacterial infection, epilepsy, depression, pain, vitamin deficiency, immune-deficiency and steroid deficiency. In another aspect, he disease is selected from the group comprising or consisting of addiction, cancer, depression, vitamin deficiency, steroid deficiency and cardiovascular diseases.

The kit can be used in the method of the invention by a layman at home. The ease of use of the kit as well as the simplicity of using the supernatant for analysis reduces the risk for errors during all steps of the method. In case of pharmaceutical active compounds, whereby frequent monitoring is important due to a narrow therapeutic window, costs for health care are reduced because no trained health care personal are needed for the monitoring. This is believed to improve compliance of the patient and thus efficacy and efficiency of the treatment. With the method of the invention there is no need for the patient to go to a health care center and there is no need for a cold chain transport to the analyzing laboratory.

In an aspect, the method or the kit is used together with an interpretation of the analysed result by a health care professional, such as a medical doctor.

In another aspect, the method or the kit as defined above is used for diagnosing a disease.

In one aspect, the method or the kit as defined above is used for diagnosing alcoholism. In another aspect, the method or the kit as defined above is used for diagnosing drug abuse.

In another aspect, the method or the kit as defined above is used for diagnosing a vitamin deficiency.

In another aspect, the method or the kit as defined above is used for monitoring an amount of API, such as tamoxifen, in blood of a patient.

In one aspect, the method or the kit as defined above is used for Tailor Dose Monitoring (TDM).

In an aspect, the method as defined above is for use in measuring or monitoring a quality and concentration of one or more pharmaceutical active compound (API) in the blood of a mammal, whereby the pharmaceutical active compound has a narrow therapeutic window selected from the group comprising or consisting of tamoxifen, digoxin, digitoxin, fosphenytoin, phenytoin, ethosuximide, alfentanil, tacrolimus, meperidine, temsirolimus, sirolimus, thiopental, fentanyl, alfentanil, theophylline, cyclosporine, clonidine, amitriptyline, protriptyline, imipramine, nortriptyline, quinidine, levothyroxine, carbamazepine, phenobarbital, ergotamine, dihydroergotamine and heparin. In another aspect the pharmaceutical active compound has a narrow therapeutic window selected from the group comprising or consisting of tamoxifen, digoxin, digitoxin, fosphenytoin, levothyroxine and carbamazepine. In another aspect the pharmaceutical active compound has a narrow therapeutic window selected from tamoxifen.

### Brief description of the drawings

The invention will now be explained more closely by the description of different embodiments of the invention and with reference to the appended figures.
Fig. 1 shows an example of a kit of parts.
Fig. 2 shows a schematic overview of the method.
Fig. 3 shows a correlation between intravenous versus capillary blood sampling.
Tables 4 to 8, listing pharmaceutical active compounds and suitable internal standards.

### Detailed description

A "narrow therapeutic window" of a pharmaceutical active compound (API)/drug is defined as a small difference in dose or blood concentration between a therapeutic effective dose/concentration and a concentration that may lead to serious therapeutic failures or adverse drug reactions. Serious events may be those, which are persistent, irreversible, slowly reversible, or life-threatening, possibly resulting in hospitalization, disability, or even death, or compounds having little difference between toxic and therapeutic doses of said compound. These types of compounds often need frequent monitoring of the concentration of the API in the blood of a mammal.

An "extraction fluid" means a solution comprising an analytical reference fluid with a known concentration of the internal standard. Preferably, said fluid can be directly used after centrifugation in an analytical instrument to measure the quality and concentration of the one or more biomarker.

An "internal standard" means a radioisotope of a biomarker in a known concentration of the biomarker in the extraction fluid.

An "analyte" means a molecule/biomarker/active pharmaceutical compound (API) present in the blood sample that will be analysed and quantified in the method.

A "radioisotope" means radioactive isotopes of an element, which are atoms that contain an unstable combination of neutrons and protons, or an excess energy in their nucleus. The radioactive isotope may be 13C or 2H (also written as "D" for deuterium, which is the same as the term "1H" used in the priority document). Further examples of suitable radioisotopes that may be incorporated include 3H (also written as "T" for tritium), 11C, 14C, 13N, 15N, 150, 170, 180, 18F, 35S, 36CI, 82Br, 75Br, 76Br, 77Br, 1231, 1241, 1251 and 1311. The radioisotope that is used will depend on the specific application of that radio-labelled derivative. In some aspects, the radioisotope is 2H. In some embodiments, the radioisotope is 3H. In some aspects, the radioisotope is 13C. In some aspects, the radionuclide is 14C. In some aspects, the radioisotope is 11C. And in some aspects, the radioisotope is 180. The internal standard may be one or more radioactive biomarkers, whereby each biomarker may have one or more radioactive isotopes.

A "mammal" means a human or an animal, such as a horse, dog, cat, cow or pig. The human may be a patient.

The term "addiction or equivalents thereof" as used herein includes addiction as a disease.

The term "disease" as used herein is meant to include disorders, illnesses and other sicknesses.

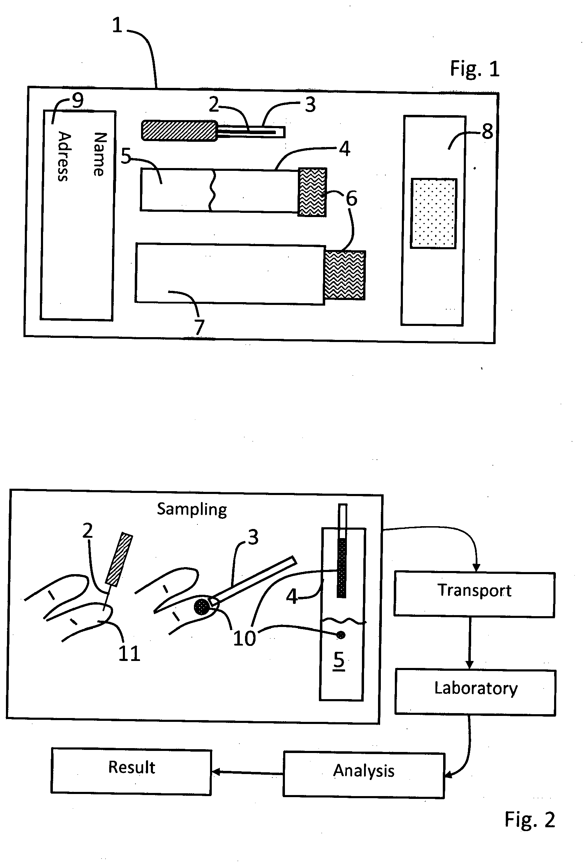

As shown in figure 1, the capillary kit 1 consists of a lancet 2, a capillary or capillary tube 3 that can take an exact volume of whole blood from 10 to 100 µl, or 50 µl and a vial 4 with a cap 6 comprising or consisting of an extraction fluid 5 with the one or more internal standard, a transport vial 7 with a cap 6, a plaster 8 and an address label 9.

The extraction fluid is optimized for the one or more specific biomarker, API or API metabolite to be measured or analyzed. Optimization is done so that the one or more biomarker is preferably stable for 7 or 14 days at room temperature, in the extraction fluid. In addition to a solvent, the extraction fluid contains an exact amount of one or more internal standard, that is, the one or more biomarker labeled with one or more radioisotope, such that only the mass of the internal standard differs from the biomarker/analyte. The one or more internal standard is adapted to assess a quality and concentration of the one or more biomarker in the blood sample.

The one or more internal standard is a radioisotope selected from a group comprising or consisting of one or more of 2H 3H, 11C, 13C, 14C, 13N, 15N, 150, 170 and 180 radioisotope. The radioisotope may be a 2H 3H, 11C, 13C and/or 14C radioisotope. The radioisotope may be a 13C radioisotope. The radioisotope may be a 2H radioisotope. The radioisotope may be a 2H and 13C radioisotope. When more than one internal standard is used a combination of different radioisotopes may be used. For example, one of the internal standards may be a 2H radioisotope for one biomarker and another internal standard may be a 13C radioisotope of a different biomarker.

If the radiolabeled molecular mass does not differ significantly from the none-radiolabeled molecular mass, the same molecule may be labeled with both 13C- and 2H-radioisotopes. For methylmalonic acid (MMA) for example, the 13C MMA- radioisotopes and the 2H-MMA-radioisotopes differ only by one atom. Therefore, the same molecule may be labeled with both 13C- and 2H-MMA- radioisotopes to improve the resolution in the analysis.

To avoid interference with endogenous MMA, a standard curve is made by a labelled 13C-MMA and the internal standard is labelled both with 13C- and 2H-MMA

As shown in figure 2, the blood sample is taken by a stick of the lancet 2 into a finger 11 giving a wound from which 10 to 100 µl of blood 10 is collected. The blood sample is immediately transferred into the vial 4 comprising or consisting of the extraction fluid 5 and one or more internal standard and shaken vigorously. The extraction fluid stops all enzyme activities in the blood 10 and an extraction of the one or more biomarker from the blood sample to the extraction fluid occurs.

The blood sample 10 will then be transported to a laboratory for analysis. The known concentration and behavior of the internal standard(s) in the analysis will immediately indicate if the sample has not been properly handled, such as too low blood volume (e.g. < 50 µl) or degradation due to heat. The quality assurance that the internal standard(s) provides for the sample is unprecedented to any other method available on the market. Which is particularly important if the sample is taken in a home environment by a layman.

When the vial 4 with the extraction fluid containing the sample arrives at the laboratory, the vial is vortexed vigorously and centrifuged. The supernatant is transferred to a tube, which can be directly used in an e.g. LC-MS/MS instrument for analysis and concentration determination of the biomarker. Optionally, an additional extraction step is performed prior to using an analytical instrument. The blood sample may be analysed using an analytical instrument, such as an instrument for mass spectrometry (MS), liquid chromatography-mass spectrometry (LC-MS, LC-MS/MS, gas chromatography-mass spectrometry (GC-MS) or GC-MS/MS. The analysis can be automized in a robotic setting.

The one or more internal standard in the extraction fluid is the same as that used in the standard curve in the analysis method. Preferably, the one or more internal standard is from the same production batch, although this is not necessary since it is possible to normalize different batches in relation to each other.

The one or more internal standard may be a biomarker for measuring a quality and concentration of any pharmaceutical active compound that can be stabilized in an extraction fluid. Examples of APIs are shown in tables 4 to 8. Suitable APIs may be selected from the group comprising tamoxifen, z-endoxifen, 4-hydroxytamoxifen, statins, steroids, such as testosterone, or vitamins, such as vitamin B or D, or compounds related to addictions, such as opioids, cocaine, heroin, fentanyl, cannabinoids, marijuana, benzodiazepines, hallucinogens and methamphetamine.

The one or more internal standard may be a biomarker for measuring a quality and concentration of a statin selected from the group comprising or consisting of atorvastatin, fluvastatin, cerivastatin, lovastatin, mevastatin, pitavastatin, pravastatin, rosuvastatin and simvastatin, or mixtures thereof.

The one or more internal standard may be a biomarker for measuring a quality and concentration of a steroid hormone, such as glucocorticoids, mineralocorticoids, androgens, oestrogens and progestogens. In one aspect, the steroid hormones is selected from the group comprising or consisting of alclometasone, prednisone, dexamethasone, triamcinolone, cortisone, fludrocortisone, oxandrolone, oxabolone, testosterone, nandrolone, diethylstilbestrol (DES) and estradiol, norethisterone, medroxyprogesterone acetate, hydroxyprogesterone caproate, cyproterone acetate, mifepristone and gestrinone, or mixtures thereof.

The one or more internal standard may be a biomarker for measuring a quality and concentration of an anti-depressant API selective serotonin reuptake inhibitors, serotoninnorepinephrine reuptake inhibitors, serotonin modulators and stimulators, serotonin antagonists and reuptake inhibitors, norepinephrine reuptake inhibitors, norepinephrinedopamine reuptake inhibitors, tricyclic antidepressants, tetracyclic antidepressants, monoamine oxidase inhibitors and NMDA receptor antagonists, or mixtures thereof.

The anti-depressant API may be selected from the group comprising or consisting of bupropiontrazodone, nefazodone, vilazodone, vortioxetine, mitriptyline, bupropion, bupropion, bupropion, bupropion, bupropion, citalopram, desvenlafaxine, duloxetine, escitalopram, fluoxetine, mirtazapine, nortriptyline, paroxetine, sertraline, trazodone and venlafaxine, or mixtures thereof.

The one or more internal standard may be a radioisotope of tamoxifen, z-endoxifen and/or 4-hydroxytamoxifen for measuring the amount of tamoxifen in blood of a patient. The API may be tamoxifen and the internal standards may be a radioisotope of tamoxifen, z-endoxifen and/or 4-hydroxytamoxifen. The API may be tamoxifen and the internal standards may be a radioisotope of tamoxifen, z-endoxifen and 4-hydroxytamoxifen. The radioisotope(s) may be a 13C radioisotope. The radioisotope(s) may be a 2H radioisotope and 13C radioisotope. Tamoxifen, z-endoxifen and 4-OH tamoxifen may all be 13C radioisotopes.

The one or more internal standard may be a radioisotope of phosphatidylethanol 16:0/18:1, which is a biomarker for measuring long-term alcohol consumption. The radioisotope may be a 2H radioisotope.

The one or more internal standard may be a radioisotope of methylmalonic acid for measuring vitamin B insufficiency. The radioisotope may be a 2H radioisotope. The radioisotope may be a 13C radioisotope. The radioisotope may be a 2H and 13C radioisotope.

The one or more internal standard may be a radioisotope of dihydrotachysterol (Vitamin D) adapted for measuring Vitamin D deficiency. The radioisotope may be a 2H radioisotope. The radioisotope may be a 13C radioisotope. The radioisotope may be a 2H and 13C radioisotope. The one or more internal standard may be a biomarker for measuring a quality and concentration of one or more pharmaceutical active compound having a small therapeutic window. The API may be selected from the group comprising or consisting of tamoxifen, digoxin, digitoxin, fosphenytoin, phenytoin, ethosuximide, alfentanil, tacrolimus, meperidine, temsirolimus, sirolimus, thiopental, fentanyl, alfentanil, theophylline, cyclosporine, clonidine, amitriptyline, protriptyline, imipramine, nortriptyline, quinidine, levothyroxine, carbamazepine, phenobarbital, ergotamine, dihydroergotamine and heparin. The pharmaceutical active compound that has a narrow therapeutic window may be selected from the group comprising or consisting of tamoxifen, digoxin, digitoxin, phosphenytoin, levothyroxine and carbamazepine. The pharmaceutical active compound may be tamoxifen.

In a reference example of the method the following step are taken;
- providing a kit of parts for sampling of blood, the kit of parts comprising a lancet and a capillary adapted to sample 50 µl blood from the human and a vial comprising an extraction fluid, which is 2-propanol or tetrahydrofuran, together with an internal standard, which is a 2H radioisotope of phosphatidylethanol 16:0/18:1,
- distributing the kit of parts to a location of the mammal,
- taking a blood sample from the mammal by creating a wound with the lancet,
- collecting a 50 µl volume of the blood in the capillary,
- entering the blood sample or the capillary into the vial,
- receiving the vial comprising the blood sample from the mammal inserted into the vial,
- optionally, extracting the fluid from the vial,
- centrifuging the vial, and
- analysing the supernatant to determine the quality of the sample and the concentration of phosphatidylethanol in the blood of the mammal.

In another reference example of the method the following step are taken;
- providing a kit of parts for sampling of blood, the kit of parts comprising a lancet and a capillary adapted to sample 50 µl blood from the human and a vial comprising an extraction fluid, which is acetonitrile, together with an internal standard, which is a 2H radioisotope of methylmalonic acid,
- distributing the kit of parts to a location of the mammal,
- taking a blood sample from the mammal by creating a wound with the lancet,
- collecting a 50 µl volume of the blood in the capillary,
- entering the blood sample or the capillary into the vial,
- receiving the vial comprising the blood sample from the mammal inserted into the vial,
- optionally, extracting the fluid from the vial,
- centrifuging the vial, and
- analysing the supernatant to determine the quality of the sample and the concentration of methylmalonic acid in the blood of the mammal.

In a further reference example of the method the following step are taken;
- providing a kit of parts for sampling of blood, the kit of parts comprising a lancet and a capillary adapted to sample 50 µl blood from the human and a vial comprising an extraction fluid, which is methanol, acetonitrile or formic acid, together with an internal standard, which is a 13C radioisotope of tamoxifen, z-endoxifen and 4-hydroxytamoxifen,
- distributing the kit of parts to a location of the mammal,
- taking a blood sample from the mammal by creating a wound with the lancet,
- collecting a 50 µl volume of the blood in the capillary,
- entering the blood sample or the capillary into the vial,
- receiving the vial comprising the blood sample from the mammal inserted into the vial,
- optionally, extracting the fluid from the vial,
- centrifuging the vial, and
- analysing the supernatant to determine the quality of the sample and the concentration of tamoxifen, z-endoxifen and 4-hydroxytamoxifen in the blood of the mammal.

In a further reference example of the method the following step are taken;
- providing a kit of parts for sampling of blood, the kit of parts comprising a lancet and a capillary adapted to sample a defined volume of blood from the human and a vial comprising an extraction fluid, which is 2-propanol, methanol, acetonitrile or formic acid, together with an internal standard, which is a 13C radioisotope of an antidepressant API (e.g. seratralin, citalpram, excitaopram, venlafaxin and flouxetin), or a statin (e.g. pravastin, rosuvastatin, simvastatin and atorvastatin) or a steroid hormone (e.g. aldosteron, androsteron, crotisol, progetsteron and testosteron),
- distributing the kit of parts to a location of the mammal,
- taking a blood sample from the mammal by creating a wound with the lancet,
- collecting a 50 µl volume of the blood in the capillary,
- entering the blood sample or the capillary into the vial,
- receiving the vial comprising the blood sample from the mammal inserted into the vial,
- optionally, extracting the fluid from the vial,
- centrifuging the vial, and
- analysing the supernatant to determine the quality of the sample and the concentration of said anti-depressant API, statin and steroid hormone (e.g. sertralin, atorvastatin or testosterone), in the blood of the mammal.

Centrifuging may be done at 16400 rpm. Analysis may be performed in an LC-MS/MS instrument. Other examples of biomarkers of interest are shown in table 1.

**Table 1**

| **Drugs** | | |
|---|---|---|
| Codeine | Ibuprofen | Dihydrocodeine |
| Morphine | Dextropropxyphene napsylate | Aminorex |
| Lidocaine | Iso-LSD | Norcocaine |
| Amitriptyline | Pemoline | Prazepam |
| Imipramine | Propafenone | Pheniramine |
| Chlorpromazine | Amphetamine sulfate | Lofexidine hydrochloride |
| Clozapine | Ecgonine methyl ester | Diphenylhydramine |
| Estazolam | 3,4-Methylenedioxy-amphetamine | Melatonin |
| Doxepin | Ketamine | Mescaline |
| Aprobarbital | Buprenorphine | Benzoylecgnine |
| Trifluoperazine | Methadone | Ecgonine ethyl ester |
| Midazolam | Fentanyl | Norketamine |
| Chlordiazepoxide | Caffeine | Hydrocodone |
| Fenfluramine | Tramadol | Lorazepam |
| Phenylpropanolamine | Flunitrazepam | |
| Amobarbital | Flurazepam | PCP |
| Barbital | Carbamazepine | Phenobarbital |
| Vancomycin | | |

Further pharmaceutical active compounds may be CPA-cyclophosphamide and its active metabolite PAM-hb, or DOC- docetaxel, DOX-doxorubicine, PAC-paclitaxel, EPI-epirubicin.

The solvent used in the extraction fluid comprises an organic solvent or a combination of two or more organic solvents. The solvents may be selected from the group comprising or consisting of 2-propanol, methanol and acetonitrile, formic acid, or mixtures thereof. For the internal standard PEth-16:0/18:1, the extraction fluid may be 2-propanol or tetrahydroferan. For the internal standard metylmalonic acid, the extraction fluid may be acetonitrile. For the internal standards tamoxifen, z-enoxifen and 4-hydroxytamoxifen, the extraction fluid may be methanol, acetonitrile, formic acid, or mixtures thereof. Table 2 lists other examples of suitable extraction fluid.

**Table 2**

| Extraction fluid | |
|---|---|
| Methanol | Chloro-butane |
| Ethanol | Ethyl-ether |
| Isopropanol | Methyl tert-butyl ether |
| Butanol | Ethanol |
| Acetonitrile | Ethyl acetate |
| Tetrahydrofuran | Acetic acid |
| Acetone | Ammonium hydroxide |
| Dichloromethane | Ammonium acetate |
| Formic acid | |

Other examples of pharmaceutical active compounds and internal standards are shown in tables 4 to 8 in the attached drawings. (Adaway JE, Therapeutic drug monitoring and LC-MS/MS, J. Chromatogr. B. 2012, 883-884, 33-49.)

The method and the kit of parts may be used for diagnosing a disease. The disease may be any disease related to the analyzed biomarker/analyte. Example of diseases may be any form of addiction or abuse, such as alcoholism, opiate abuse, or cancer, such as breast cancer, prostate cancer, or cardiovascular diseases, such as high blood pressure, or steroid deficiency diseases.

The method and the kit of parts may be used for Tailor Dose Monitoring (TDM).

The method and the kit of parts may be used for diagnosis and/or monitoring drug abuse or alcoholism.

### Example 1, Method of measuring long term alcohol use and validation of the method.

The kit as defined above was used. The kit comprised a vial that contained 200 µl of extraction solution 80% isopropanol with 20% tetrahydrofuran and 0.1 µM D5-PEth 16:0/18:1 (i.e. 2H radioisotope of phosphatidylethanol 16:0/18:1).
50 µl blood was collected from human volunteers using the lancet and added to the extraction solution in the vial. Or 50 µl blood was collected from human volunteers using venous blood. The blood from seven different human volunteers was collected.

The vials were shaken and transported to the laboratory for analysis.

The vials were vortexed and centrifuged. 20 µl of the supernatant was directly injected into the LC-MS/MS system. The LC-MS/MS system contains: Mobile phase A; 20% acetonitrile, 0,5 mM ammonia and 1 mM acetic acid in water, mobile phase B; 20% acetonitrile, 20 % tetrahydrofuran and 60% 2-propanol were used.

The test showed that the same results were obtained independent how the blood samples were collected from the human volunteers.

The analysis allows for the quantification of low levels of 0.005 mmol/ml.

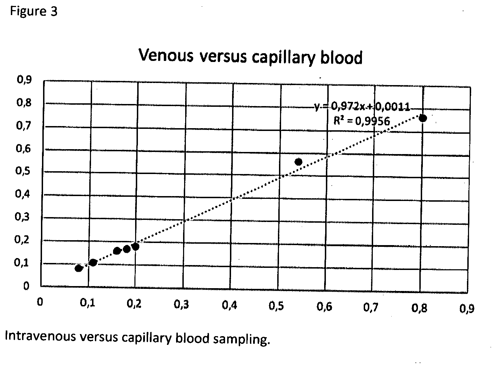

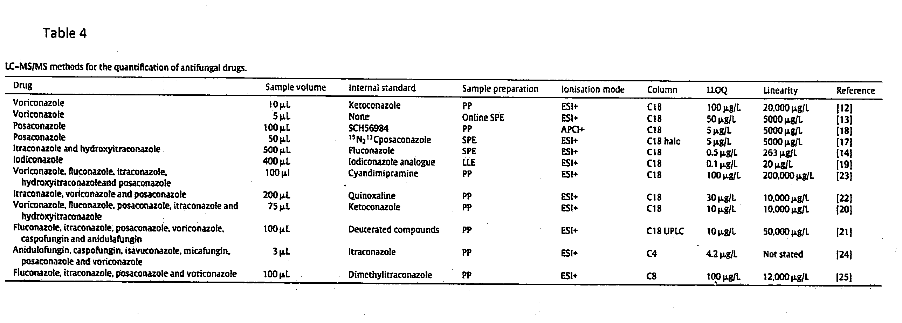

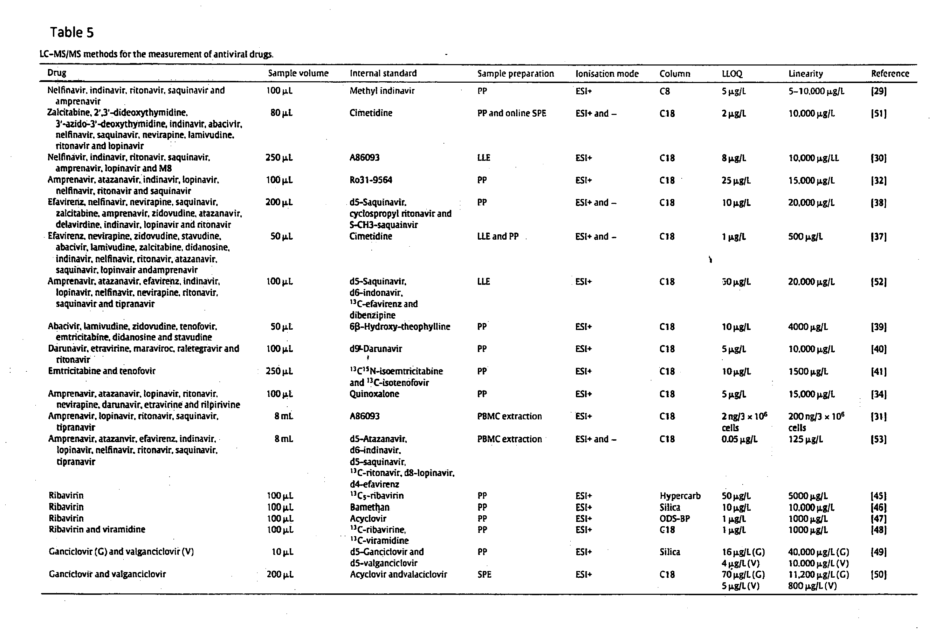

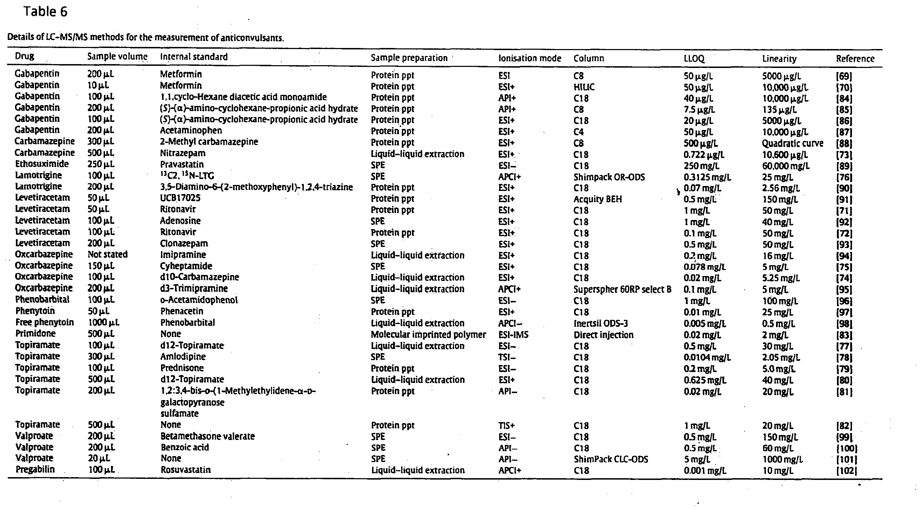

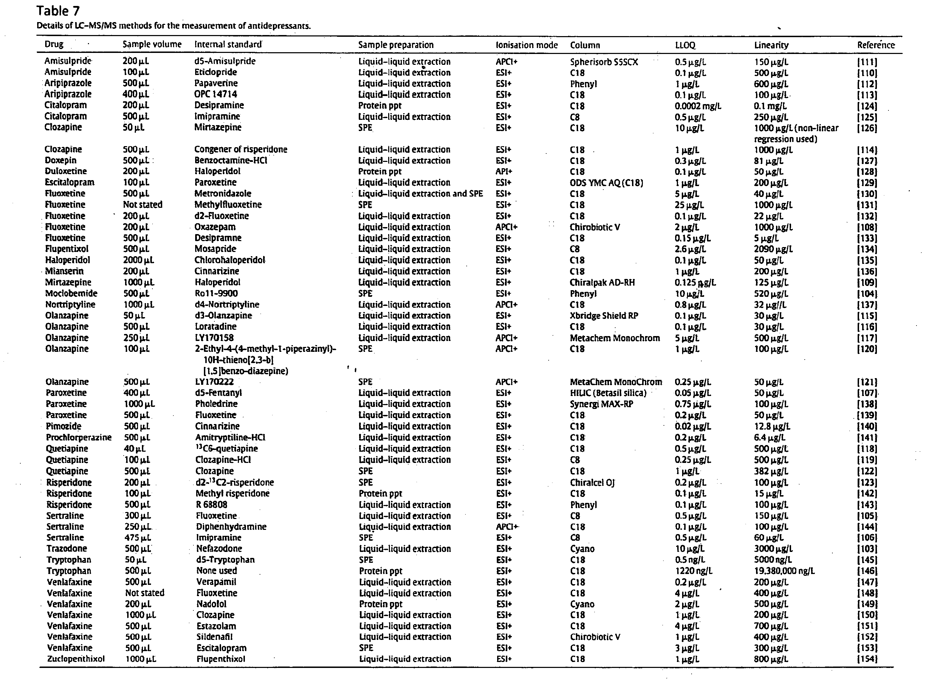

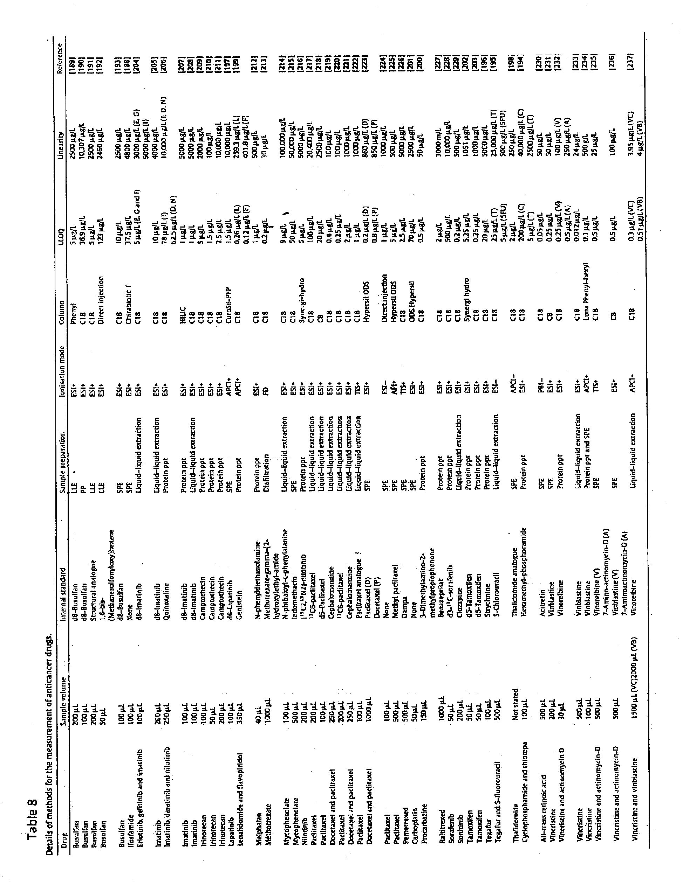

The results in table 3 and figure 3 show that there are no differences between a venous blood sample and a sample taken by lancet in the finger.

**Table 3**

| Individual | Conc. | %CV |
|---|---|---|
| 1 | 0,16 | 7 |
| 2 | 0,78 | 4 |
| 3 | 0,55 | 3 |
| 1 | 0,18 | 4 |
| 3 | 0,52 | 4 |
| 5 | 0,19 | 5 |
| 6 | 0,11 | 7 |
| 7 | 0,08 | 3 |
| 8 | 0,18 | 2 |

Intravenous vs. capillary blood sampling; The concentration of triplicates from an intravenous sample and capillary blood sampled in triplicates are compared. Samples are collected from 7 healthy individuals, in a total of nine sample extractions, at two different days.

### Example 2, Monitoring amount of tamoxifen in blood of a patient

The kit as defined above was used. The kit comprised a vial that contained 150 µl extraction solution containing acetonitrile with 0.2 % formic acid and 0,1 ng/mL each of 13C-labeled tamoxifen, 13C-labeled tamoxifen, 13C-labeled z-endoxifen and 13C-labeled 4-OH tamoxifen, 0.1 µM of each. 50 µl of blood from the patient was collected using the lancet. Or 50 µl blood was collected from human volunteers using venous blood. The blood was added to 150 µl extraction solution.

The vial was shaken and transported to the laboratory for analysis.

The vial was vortexed and centrifuged. 150 µl of the supernatant was collected and transferred to glass vials. The protein precipitation solution was evaporated to dryness and reconstituted in 60 µl 20% acetonitrile in water. 20 µl of the solution was injected into the LC-MS/MS system. The LC-MS/MS system: the mobile phases A; 0,1 % formic acid in water and mobile phase B; 0,1 % formic acid in acetonitrile.

Three validation batches were prepared and analyzed at three different occasions. Each validation batch comprised two sets of all calibration standards, two sets of all quality control samples and a number of blank samples.

The results show that there are no differences between a venous blood sample and a sample taken by a lancet in the finger.

### Example 3, Monitoring amount of methylmalonic acid in blood of a patient

The kit comprised a vial that contained 100 µl extraction solution containing 0.5 µmol/L labelled MMA in water. 50 µl of blood from the patient was collected using the lancet. Or 50 µl blood was collected from human volunteers using venous blood. The blood was added to 100 µl extraction solution.

The vial was shaken and transported to the laboratory for analysis.

The vial was vortexed and transferred to a solid phase extraction (SPE) column. The column was eluted by 150 µl 3% formic acid in acetonitrile. The eluate was evaporated to dryness. 100 µl water was added, 20 µl was transferred to the LC-MS/MS system.

The mobile phases A: 0.05 % acetic acid in 90 % acetonitrile in water and mobile phase B: 0.5 % formic acid in 20 % acetonitrile in water.

The results show that there are no differences between a venous blood sample and a sample taken by a lancet in the finger

## Claims

1. A method for measuring a concentration of one or more biomarker/analyte in blood from a mammal comprising:
- providing a kit of parts for sampling blood, the kit of parts comprising a lancet and a capillary adapted to sample a defined volume of blood from the mammal and a vial comprising an extraction fluid together with one or more internal standard, wherein the one or more internal standard is a radioisotope of one or more biomarker selected from a group comprising one or more of 2H, 3H, 11C, 13C, 14C, 13N, 15N, 150, 170 and 180 radioisotope of said biomarker adapted to assess a quality and a concentration of the one or more biomarker in the blood sample,
- distributing the kit of parts to a location of the mammal,
- receiving the vial comprising a blood sample from the mammal inserted into the vial,
- analysing the blood sample to determine the quality of the sample and the concentration of the one or more biomarker in the blood of the mammal by centrifuging the vial and performing a direct analysis of the supernatant, and normalizing the analyzed results between different samples and a standard curve.

2. The method according to claim 1, wherein the one or more internal standard is a 2H, 3H, 11C, 13C, 14C radioisotope of one or more biomarker.

3. The method according to claim 1, wherein the one or more internal standard is a 13C and/or 2H radioisotope of one or more biomarker.

4. The method according to any one of the preceding claims, wherein the one or more internal standard is adapted for measuring a quality and a concentration of one or more pharmaceutical active compound (API) that can be stabilized in an extraction fluid, whereby the API is selected from the group comprising antifungal API, antiviral API, immunodepression API, anticonvulsant API, antidepressant API, antibiotic API, anticancer API, vitamins, cardiovascular API, painkilling API and opiates.

5. The method according to any one of claims 1 to 3, wherein the one or more internal standard is adapted for measuring or monitoring a quality and a concentration of one or more pharmaceutical active compound (API) in the blood of a mammal that can be stabilized in an extraction fluid, whereby the one or more API is selected from the group comprising tamoxifen, 4-hydroxytamoxifen, CPA-cyclophosphamide and its active metabolite PAM-hb, DOC-docetaxel, DOX-doxorubicine, PAC-paclitaxel, EPI-epirubicin, statins, steroids, such as testosterone, or vitamins, such as vitamin B or D, or compounds related to addictions, such as opioids, cocaine, heroin, fentanyl, cannabinoids, marijuana, benzodiazepines, hallucinogens and methamphetamine, codeine, ibuprofen, dihydrocodeine, morphine, dextropropoxyphene napsylate, aminorex, lidocaine, iso-LSD, norcocaine, amitriptyline, pemoline, prazepam, imipramine, propafenone, pheniramine, chlorpromazine, amphetamine sulfate, lofexidine hydrochloride, clozapine, ecgonine methyl ester, diphenylhydramine, estazolam, 3,4-methylenedioxy-amphetamine, melatonin, doxepin, ketamine, mescaline, aprobarbital, buprenorphine, benzoylecgnine, trifluoperazine, methadone, ecgonine ethyl ester, midazolam, fentanyl, norketamine, chlordiazepoxide, caffeine, hydrocodone, fenfluramine, tramadol, lorazepam, phenylpropanolamine, flunitrazepam, 2C-B, amobarbital, flurazepam, phencyclidine (PCP), barbital, carbamazepine, vancomycin and phenobarbital.

6. The method according to any one of claims 1 to 3, wherein the internal standard is a 13C-radioisotope of tamoxifen, z-endoxifen and 4-hydroxytamoxifen.

7. The method according to any one of claims 1 to 3, wherein the one or more internal standard is phosphatidylethanol 16:0/18:1 (PEth-16:0/18:1, PEth 16:0/18:2, PEth 18:1/16:0, and/or PEth 18:2/16:0) adapted for measuring long-term alcohol consumption.

8. The method according to any one of claims 1 to 3, wherein the one or more internal standard is a radioisotope of methylmalonic acid adapted for measuring B-vitamin deficiency in a mammal or, wherein the one or more internal standard is a radioisotope of dihydrotachysterol adapted for measuring D-vitamin deficiency in a mammal.

9. The method according to any one of claims 1 to 3, wherein the one or more internal standard is adapted for measuring or monitoring a quality and a concentration of one or more pharmaceutical active compound (API) in the blood of a mammal that can be stabilized in an extraction fluid, whereby the one or more API is selected from the group comprising compounds related to addictions, such as opioids, cocaine, heroin, fentanyl, cannabinoids, marijuana, benzodiazepines, hallucinogens and methamphetamine, amphetamine, codeine, dihydrocodeine, morphine, iso-LSD, phencyclidine (PCP), norcocaine, fentanyl, methadone, hydrocodone and tramadol.

10. The method according to any one of claims 1 to 3, wherein the one or more internal standard is adapted for measuring or monitoring a quality and a concentration of one or more statins selected from the group comprising atorvastatin, fluvastatin, cerivastatin, lovastatin, mevastatin, pitavastatin, pravastatin, rosuvastatin and simvastatin, or mixtures thereof.

11. The method according to any one of claims 1 to 3, wherein the one or more internal standard is adapted for measuring or monitoring a quality and a concentration of one or more steroid hormones selected from the group comprising alclometasone, prednisone, dexamethasone, triamcinolone, cortisone, fludrocortisone, oxandrolone, oxabolone, testosterone, nandrolone, diethylstilbestrol (DES) and estradiol, norethisterone, medroxyprogesterone acetate, hydroxyprogesterone caproate, cyproterone acetate, mifepristone and gestrinone, or mixtures thereof.

12. The method according to any one of claims 1 to 3, wherein the one or more internal standard is adapted for measuring or monitoring a quality and a concentration of one or more antidepressant API selected from the group comprising bupropiontrazodone, nefazodone, vilazodone, vortioxetine, mitriptyline, bupropion, citalopram, desvenlafaxine, duloxetine, escitalopram, fluoxetine, mirtazapine, nortriptyline, paroxetine, sertraline, trazodone, venlafaxine, or mixtures thereof.

13. The method according to any one of the preceding claims, wherein the extraction fluid is selected from the group comprising 2-propanol, methanol and acetonitrile, formic acid or mixtures thereof, which fluid is adapted to substantially stop all enzyme activity in the blood sample, stabilize the sample and extract the biomarker from the blood sample.

14. The method according to any one of the preceding claims for use in measuring or monitoring a quality and a concentration of one or more active pharmaceutical compound in the blood of a mammal, whereby the pharmaceutical active compound has a narrow therapeutic window selected from the group comprising or consisting of tamoxefin, digoxin, digitoxin, fosphenytoin, phenytoin, ethosuximide, alfentanil, tacrolimus, meperidine, temsirolimus, sirolimus, thiopental, fentanyl, alfentanil, theophylline, cyclosporine, clonidine, amitriptyline, protriptyline, imipramine, nortriptyline, quinidine, levothyroxine, carbamazepine, phenobarbital, ergotamine, dihydroergotamine and heparin.

15. The method according to any one of claims 1 to 14, for use in Tailor Dose Monitoring (TDM).

## Patentansprüche

1. Verfahren zum Messen einer Konzentration eines oder mehrerer Biomarker/Analyten in Blut von einem Säugetier, umfassend:
- Bereitstellen eines Kits aus Teilen zur Probenahme von Blut, wobei das Kit aus Teilen eine Lanzette und ein Kapillaröhrchen umfasst, die auf die Probenahme eines definierten Blutvolumens von dem Säugetier ausgelegt sind, und ein Fläschchen, das ein Extraktionsfluid zusammen mit einem oder mehreren internen Standard umfasst, wobei der eine oder die mehreren interne Standard ein Radioisotop eines oder mehrerer Biomarker ist, ausgewählt aus einer Gruppe, umfassend eines oder mehrere von 2H-, 3H-, 11C-, 13C-, 14C-, 13N-, 15N-, 150-,170- und 18O-Radioisotop des Biomarkers, die darauf ausgelegt sind, eine Qualität und eine Konzentration des einen oder der mehreren Biomarker in der Blutprobe zu bewerten,
- Verteilen des Kits aus Teilen an eine Stelle des Säugetiers,
- Aufnehmen des Fläschchens, umfassend eine in das Fläschchen eingeführte Blutprobe von dem Säugetier,
- Analysieren der Blutprobe, um die Qualität der Probe und die Konzentration des einen oder der mehreren Biomarker in dem Blut des Säugetiers durch Zentrifugieren des Fläschchens und Durchführen einer direkten Analyse des Überstands zu bestimmen und Normalisieren der analysierten Ergebnisse zwischen verschiedenen Proben und einer Standardkurve.

2. Verfahren nach Anspruch 1, wobei der eine oder die mehreren interne Standard ein 2H-, 3H-, 11C-, 13C-, 14C-Radioisotop eines oder mehrerer Biomarker ist.

3. Verfahren nach Anspruch 1, wobei der eine oder die mehreren interne Standard ein 13C- und/oder 2H-Radioisotop eines oder mehrerer Biomarker ist.

4. Verfahren nach einem der vorstehenden Ansprüche, wobei der eine oder die mehreren interne Standard zum Messen einer Qualität und einer Konzentration einer oder mehrerer pharmazeutisch aktiven Verbindung (API) angepasst ist, die in einem Extraktionsfluid stabilisiert werden kann, wobei die API ausgewählt ist aus der Gruppe, umfassend antimykotische API, antivirale API, Immunodepression-API, Antikonvulsivum-API, Antidepressivum-API, antibiotische API, Anti-Krebs-API, Vitamine, Herz-Kreislauf-API, schmerzstillende API und Opiate.

5. Verfahren nach einem der Ansprüche 1 bis 3, wobei der eine oder die mehreren interne Standard zum Messen oder Überwachen einer Qualität und einer Konzentration einer oder mehrerer pharmazeutisch aktiven Verbindung (API) in dem Blut eines Säugetiers angepasst ist, die in einem Extraktionsfluid stabilisiert werden kann, wobei die eine oder mehreren API ausgewählt ist aus der Gruppe, umfassend Tamoxifen, 4-Hydroxytamoxifen, CPA-Cyclophosphamid und seinem aktiven Metaboliten PAM-hb, DOC-Docetaxel, DOX-Doxorubicin, PAC-Paclitaxel, EPI-Epirubicin, Statine, Steroide wie Testosteron oder Vitamine wie Vitamin B oder D oder Verbindungen, die sich auf Abhängigkeiten beziehen, wie Opioide, Kokain, Heroin, Fentanyl, Cannabinoide, Marihuana, Benzodiazepine, Halluzinogene und Methamphetamin, Codein, Ibuprofen, Dihydrocodein, Morphin, Dextropropoxyphennapsylat, Aminorex, Lidocain, Iso-LSD, Norkokain, Amitriptylin, Pemolin, Prazepam, Imipramin, Propafenon, Pheniramin, Chlorpromazin, Amphetaminsulfat, Lofexidinhydrochlorid, Clozapin, Ecgoninmethylester, Diphenylhydramin, Estazolam, 3,4-Methylendioxyamphetamin, Melatonin, Doxepin, Ketamin, Mescalin, Aprobarbital, Buprenorphin, Benzoylecgnin, Trifluoperazin, Methadon, Ecgoninethylester, Midazolam, Fentanyl, Norketamin, Chlordiazepoxid, Koffein, Hydrocodon, Fenfluramin, Tramadol, Lorazepam, Phenylpropanolamin, Flunitrazepam, 2C-B, Amobarbital, Flurazepam, Phencyclidin (PCP), Barbital, Carbamazepin, Vancomycin und Phenobarbital.

6. Verfahren nach einem der Ansprüche 1 bis 3, wobei der interne Standard ein 13C-Radioisotop von Tamoxifen, z-Endoxifen und 4-Hydroxytamoxifen ist.

7. Verfahren nach einem der Ansprüche 1 bis 3, wobei der eine oder die mehreren interne Standard Phosphatidylethanol 16:0/18:1 (PEth-16:0/18:1, PEth 16:0/18:2, PEth 18:1/16:0 und/oder PEth 18:2/16:0), angepasst zum Messen von Langzeitalkoholkonsum, ist.

8. Verfahren nach einem der Ansprüche 1 bis 3, wobei der eine oder die mehreren interne Standard ein Radioisotop von Methylmalonsäure ist, angepasst zum Messen von B-Vitamin-Mangel bei einem Säugetier, oder wobei der eine oder die mehreren interne Standard ein Radioisotop von Dihydrotachysterol ist, angepasst zum Messen von D-Vitamin-Mangel bei einem Säugetier.

9. Verfahren nach einem der Ansprüche 1 bis 3, wobei der eine oder die mehreren interne Standard zum Messen oder Überwachen einer Qualität und einer Konzentration einer oder mehrerer pharmazeutisch aktiven Verbindung (API) in dem Blut eines Säugetiers angepasst ist, die in einem Extraktionsfluid stabilisiert werden kann, wobei die eine oder mehreren API ausgewählt ist aus der Gruppe, umfassend Verbindungen, die sich auf Abhängigkeiten beziehen, wie Opioide, Kokain, Heroin, Fentanyl, Cannabinoide, Marihuana, Benzodiazepine, Halluzinogene und Methamphetamin, Amphetamin, Codein, Dihydrocodein, Morphin, Iso-LSD, Phencyclidin (PCP), Norkokain, Fentanyl, Methadon, Hydrocodon und Tramadol.

10. Verfahren nach einem der Ansprüche 1 bis 3, wobei der eine oder die mehreren interne Standard zum Messen oder Überwachen einer Qualität und einer Konzentration von einem oder mehreren Statinen angepasst ist, ausgewählt aus der Gruppe, umfassend Atorvastatin, Fluvastatin, Cerivastatin, Lovastatin, Mevastatin, Pitavastatin, Pravastatin, Rosuvastatin und Simvastatin oder Mischungen davon.

11. Verfahren nach einem der Ansprüche 1 bis 3, wobei der eine oder die mehreren interne Standard zum Messen oder Überwachen einer Qualität und einer Konzentration eines oder mehrerer Steroidhormone angepasst ist, ausgewählt aus der Gruppe, umfassend Alclometason, Prednison, Dexamethason, Triamcinolon, Cortison, Fludrocortison, Oxandrolon, Oxabolon, Testosteron, Nandrolon, Diethylstilbestrol (DES) und Östradiol, Norethisteron, Medroxyprogesteronacetat, Hydroxyprogesteroncaproat, Cyproteronacetat, Mifepriston und Gestrinon oder Mischungen davon.

12. Verfahren nach einem der Ansprüche 1 bis 3, wobei der eine oder die mehreren interne Standard zum Messen oder Überwachen einer Qualität und einer Konzentration einer oder mehrerer Antidepressivum-API angepasst ist, ausgewählt aus der Gruppe, umfassend Bupropiontrazodon, Nefazodon, Vilazodon, Vortioxetin, Mitriptylin, Bupropion, Citalopram, Desvenlafaxin, Duloxetin, Escitalopram, Fluoxetin, Mirtazapin, Nortriptylin, Paroxetin, Sertralin, Trazodon, Venlafaxin oder Mischungen davon.

13. Verfahren nach einem der vorstehenden Ansprüche, wobei das Extraktionsfluid ausgewählt ist aus der Gruppe, umfassend 2-Propanol, Methanol und Acetonitril, Ameisensäure oder Mischungen davon, wobei das Fluid angepasst ist, um im Wesentlichen die gesamte Enzymaktivität in der Blutprobe zu stoppen, die Probe zu stabilisieren und den Biomarker aus der Blutprobe zu extrahieren.

14. Verfahren nach einem der vorstehenden Ansprüche zur Verwendung beim Messen oder Überwachen einer Qualität und einer Konzentration einer oder mehrerer aktiven pharmazeutischen Verbindung in dem Blut eines Säugetiers, wobei die pharmazeutische aktive Verbindung ein schmales therapeutisches Fenster aufweist, ausgewählt aus der Gruppe, umfassend oder bestehend aus Tamoxefin, Digoxin, Digitoxin, Fosphenytoin, Phenytoin, Ethosuximid, Alfentanil, Tacrolimus, Meperidin, Temsirolimus, Sirolimus, Thiopental, Fentanyl, Alfentanil, Theophyllin, Cyclosporin, Clonidin, Amitriptylin, Protriptylin, Imipramin, Nortriptylin, Chinidin, Levothyroxin, Carbamazepin, Phenobarbital, Ergotamin, Dihydroergotamin und Heparin.

15. Verfahren nach einem der Ansprüche 1 bis 14 zur Verwendung bei Tailor Dose Monitoring (TDM).

## Revendications

1. Procédé pour la mesure d'une concentration d'un ou plusieurs biomarqueurs/analytes dans le sang d'un mammifère comprenant :
- la fourniture d'une trousse de pièces pour l'échantillonnage de sang, la trousse de pièces comprenant une lancette et un capillaire adaptés pour échantillonner un volume de sang défini du mammifère et un flacon comprenant un fluide d'extraction conjointement avec un ou plusieurs étalons internes, dans lequel les un ou plusieurs étalons internes sont un radio-isotope d'un ou plusieurs biomarqueurs choisis dans un groupe comprenant un ou plusieurs des radioisotopes 2H, 3H, 11C, 13C, 14C, 13N, 15N, 150, 170 et 180 dudit biomarqueur adaptés pour évaluer une qualité et une concentration des un ou plusieurs biomarqueurs dans l'échantillon de sang,
- la distribution du kit de pièces à un emplacement du mammifère,
- la réception du flacon comprenant un échantillon de sang du mammifère inséré dans le flacon,
- l'analyse de l'échantillon de sang pour déterminer la qualité de l'échantillon et la concentration de l'un ou plusieurs biomarqueurs dans le sang du mammifère par centrifugation du flacon et réalisation d'une analyse directe du surnageant, et la normalisation des résultats analysés entre différents échantillons et une courbe étalon.

2. Procédé selon la revendication 1, dans lequel les uns ou plusieurs étalons internes sont un radio-isotope 2H, 3H, 11C, 13C, 14C d'un ou plusieurs biomarqueurs.

3. Procédé selon la revendication 1, dans lequel les un ou plusieurs étalons internes sont un radio-isotope 13C et/ou 2H d'un ou plusieurs biomarqueurs.

4. Procédé selon l'une quelconque des revendications précédentes, dans lequel les un ou plusieurs étalons internes sont adaptés pour la mesure d'une qualité et d'une concentration d'un ou plusieurs composés actifs pharmaceutiques (API) qui peuvent être stabilisés dans un fluide d'extraction, moyennant quoi l'API est choisi dans le groupe comprenant un API antifongique, un API antiviral, un API d'immunodépression, un API anticonvulsif, un API antidépresseur, un API antibiotique, un API anticancéreux, des vitamines, un API cardiovasculaire, un API antidouleur, et des opiacées.

5. Procédé selon l'une quelconque des revendications 1 à 3, dans lequel les uns ou plusieurs étalons internes sont adaptés pour la mesure ou la surveillance d'une qualité et d'une concentration d'un ou plusieurs composés actifs pharmaceutiques (API) dans le sang d'un mammifère qui peuvent être stabilisés dans un fluide d'extraction, moyennant quoi les uns ou plusieurs API sont choisis dans le groupe comprenant le tamoxifène, le 4-hydroxytamoxifène, le CPA-cyclophosphamide et son métabolite actif PAM-hb, le DOC-docétaxel, la DOX-doxorubicine, le PAC-paclitaxel, l'EPI-épirubicine, les statines, les stéroïdes, tels que la testostérone, ou des vitamines, telles que la vitamine B ou D, ou des composés liés à des addictions, tels que les opioïdes, la cocaïne, l'héroïne, le fentanyl, les cannabinoïdes, la marijuana, les benzodiazépines, les hallucinogènes et la méthamphétamine, la codéine, l'ibuprofène, la dihydrocodéine, la morphine, le napsylate de dextropropoxyphène, l'aminorex, la lidocaïne, l'iso-LSD, la norcocaïne, l'amitriptyline, la pémoline, le prazépam, l'imipramine, la propafénone, la phéniramine, la chlorpromazine, le sulfate d'amphétamine, le chlorhydrate de lofexidine, la clozapine, l'ester méthylique d'ecgonine, la diphénylhydramine, l'estazolam, la 3,4-méthylènedioxy-amphétamine, la mélatonine, la doxépine, la kétamine, la mescaline, l'aprobarbital, la buprénorphine, la benzoylecgnine, la trifluopérazine, la méthadone, l'ester éthylique d'ecgonine, le midazolam, le fentanyl, la norkétamine, le chlordiazépoxide, la caféine, l'hydrocodone, la fenfluramine, le tramadol, le lorazépam, la phénylpropanolamine, le flunitrazépam, le 2C-B, l'amobarbital, le flurazépam, la phéncyclidine (PCP), le barbital, la carbamazépine, la vancomycine et le phénobarbital.

6. Procédé selon l'une quelconque des revendications 1 à 3, dans lequel l'étalon interne est un radio-isotope 13C du tamoxifène, du z-endoxifène et du 4-hydroxytamoxifène.

7. Procédé selon l'une quelconque des revendications 1 à 3, dans lequel les un ou plusieurs étalons internes sont le phosphatidyléthanol 16:0/18:1 (PEth-16:0/18:1, PEth 16:0/18:2, PEth 18:1/16:0, et/ou PEth 18:2/16:0), adapté pour la mesure de la consommation d'alcool à long terme.

8. Procédé selon l'une quelconque des revendications 1 à 3, dans lequel les un ou plusieurs étalons internes sont un radio-isotope d'acide méthylmalonique adapté pour la mesure d'une carence en vitamine B chez un mammifère ou, dans lequel les un ou plusieurs étalons internes sont un radio-isotope de dihydrotachystérol adapté pour la mesure d'une carence en vitamine D chez un mammifère.

9. Procédé selon l'une quelconque des revendications 1 à 3, dans lequel les un ou plusieurs étalons internes sont adaptés pour la mesure ou la surveillance d'une qualité et d'une concentration d'un ou plusieurs composés actifs pharmaceutiques (API) dans le sang d'un mammifère qui peuvent être stabilisés dans un fluide d'extraction, moyennant quoi les uns ou plusieurs API sont choisis dans le groupe comprenant des composés liés à des addictions, tels que les opioïdes, la cocaïne, l'héroïne, le fentanyl, les cannabinoïdes, la marijuana, les benzodiazépines, les hallucinogènes et la méthamphétamine, l'amphétamine, la codéine, la dihydrocodéine, la morphine, l'iso-LSD, la phéncyclidine (PCP), la norcocaïne, le fentanyl, la méthadone, l'hydrocodone et le tramadol.

10. Procédé selon l'une quelconque des revendications 1 à 3, dans lequel les un ou plusieurs étalons internes sont adaptés pour la mesure ou la surveillance d'une qualité et d'une concentration d'une ou plusieurs statines choisies dans le groupe comprenant l'atorvastatine, la fluvastatine, la cérivastatine, la lovastatine, la mévastatine, la pitavastatine, la pravastatine, la rosuvastatine et la simvastatine, ou des mélanges de celles-ci.

11. Procédé selon l'une quelconque des revendications 1 à 3, dans lequel les un ou plusieurs étalons internes sont adaptés pour la mesure ou la surveillance d'une qualité et d'une concentration d'une ou plusieurs hormones stéroïdiennes choisies dans le groupe comprenant l'alclométasone, la prednisone, la dexaméthasone, la triamcinolone, la cortisone, la fludrocortisone, l'oxandrolone, l'oxabolone, la testostérone, la nandrolone, le diéthylstilbestrol (DES) et l'œstradiol, la noréthistérone, l'acétate de médroxyprogestérone, le caproate d'hydroxyprogestérone, l'acétate de cyprotérone, le mifépristone et la gestrinone, ou des mélanges de ceux-ci.

12. Procédé selon l'une quelconque des revendications 1 à 3, dans lequel les un ou plusieurs étalons internes sont adaptés pour la mesure ou la surveillance d'une qualité et d'une concentration d'un ou plusieurs API antidépresseurs choisis dans le groupe comprenant la bupropiontrazodone, la néfazodone, la vilazodone, la vortioxétine, la mitriptyline, le bupropion, le citalopram, la desvenlafaxine, la duloxétine, l'escitalopram, la fluoxétine, la mirtazapine, la nortriptyline, la paroxétine, la sertraline, la trazodone, la venlafaxine, ou des mélanges de ceux-ci.

13. Procédé selon l'une quelconque des revendications précédentes, dans lequel le fluide d'extraction est choisi dans le groupe comprenant le 2-propanol, le méthanol et l'acétonitrile, l'acide formique ou des mélanges de ceux-ci, lequel fluide est adapté pour arrêter sensiblement toute activité enzymatique dans l'échantillon de sang, stabiliser l'échantillon et extraire le biomarqueur de l'échantillon de sang.

14. Procédé selon l'une quelconque des revendications précédentes pour une utilisation dans la mesure ou la surveillance d'une qualité et d'une concentration d'un ou plusieurs composés pharmaceutiques actifs dans le sang d'un mammifère, moyennant quoi le composé actif pharmaceutique a une fenêtre thérapeutique étroite choisie dans le groupe comprenant ou constitué par la tamoxéfine, la digoxine, la digitoxine, la fosphénytoïne, la phénytoïne, l'éthosuximide, l'alfentanil, le tacrolimus, la mépéridine, le temsirolimus, le sirolimus, le thiopental, le fentanyl, l'alfentanil, la théophylline, la cyclosporine, la clonidine, l'amitriptyline, la protriptyline, l'imipramine, la nortriptyline, la quinidine, la lévothyroxine, la carbamazépine, le phénobarbital, l'ergotamine, la dihydroergotamine et l'héparine.

15. Composé selon l'une quelconque des revendications 1 à 14, pour une utilisation dans une surveillance de dose personnalisée (TDM).
